Europäisches Patentamt

European Patent Office

Office européen des brevets

(11) Publication number: **0 353 920**
**A2**

# (12) EUROPEAN PATENT APPLICATION

(21) Application number: **89307468.2**

(22) Date of filing: **21.07.89**

(51) Int. Cl.⁴: **C07C 29/15 , C07C 31/04 , C07C 31/08 , C07C 31/10**

(30) Priority: **01.08.88 US 226902**

(43) Date of publication of application:
**07.02.90 Bulletin 90/06**

(84) Designated Contracting States:
**DE FR GB IT NL**

(71) Applicant: **TEXACO DEVELOPMENT CORPORATION**
**2000 Westchester Avenue**
**White Plains New York 10650(US)**

(72) Inventor: **Prada-Silva, Guillermo**
**4 Denim Drive**
**Wappinger Falls, N. Y. 12590(US)**
Inventor: **Sanford, Gerald Glen**
**P.O.Box 402**
**Glenham, N. Y. 1252(US)**
Inventor: **Wong, Suk-Fun**
**14A Route 216**
**Stormville, N.Y. 12582(US)**
Inventor: **Storm, David Anthony**
**35 Valley View Terrace**
**Montvale, N.J. 07645(US)**

(74) Representative: **Brock, Peter William et al**
**URQUHART-DYKES & LORD 91 Wimpole Street**
**London W1M 8AH(GB)**

(54) Slurry-phase process to increase yield of mixed alcohols from synthesis gas.

(57) The yield of alcohols produced by reacting synthesis gas (carbon monoxide and hydrogen, over a catalyst can be improved by forming the catalyst into a slurry with an inert liquid medium, such as an oil having paraffinic and naphthenic components.

EP 0 353 920 A2

## SLURRY-PHASE PROCESS TO INCREASE YIELD OF MIXED ALCOHOLS FROM SYNTHESIS GAS

This invention relates to a novel method for converting synthesis gas to a mixture of lower aliphatic alcohols and, more particularly, to an improved process in which the catalyst is slurried in an inert liquid phase to produce a mixture of lower aliphatic alcohols from syngas.

Lower aliphatic alcohols have been proposed as fuel extenders for gasoline for fueling internal combustion engines. Certain mixtures of lower aliphatic alcohols for use in motor fuels have the EPA approval and are currently being marketed in the United States. The lower aliphatic alcohols can be produced from a domestically available non-petroleum source and their use in fuels serves to lessen dependence on imported crude oil or petroleum products.

Hydrogen and carbon monoxide, or a synthesis gas mixture of same, can be reacted to form lower aliphatic alcohols. A synthesis gas feedstream can be produced from non-petroleum sources, such as coal, biomass or other hydrocarbonaceous materials. The synthesis gas mixture can be produced in a partial oxidation reaction of the hydrocarbonaceous material as, for example, in a coal gasification process.

Numerous catalytic processes have been studied in attempts to provide a viable process for the production of aliphatic alcohols from synthesis gas or from a mixture of hydrogen and carbon monoxide. In these process as, however, the catalytic synthesis of alcohols has been conventionally carried out in two-phase reactors with the syngas and products in the vapor-phase and the catalyst in the solid phase. Due to the exothermicity of the reactions involved and the low heat capacity of the vapor, the potential for thermal runaway and damage to the catalyst is considerable in two-phase systems.

Chem Systems conceived the idea of a Liquid Phase Methanol Process (LPMEOH) in the mid-1970's. They proposed addition of an inert liquid oil to remove the heat of reaction and allow better temperature control. The Chem Systems work, however, was solely directed to the production of methanol. In contrast to this, the present process is directed to a slurry-phase method for producing an alcohol mixture containing a substantial amount of aliphatic alcohols having from 2 to 6 carbon atoms.

U.S. Patent 3,345,427 discloses a dehydrogenation catalyst and process in which the catalyst consists of nickel, molybdenum and alkaline metal oxides on an alumina support.

U.S. Patent 4,096,164 discloses a process for reacting hydrogen and carbon monoxide in the presence of a solid catalyst comprising rhodium with molybdenum or tungsten to product two carbon-atom oxygenated hydrocarbons in which ethanol is the major component.

U.S. Patent 4,243,553 and U.S. Patent 4,243,554 disclose a molybdenum desulfide catalyst that is useful in the water gas shift, methanation, hydrogenation and dehydrogenation processes. US-A-4607056 and US-A-4607055 disclose synthesis gas to alcohol processes in which the catalyst comprises molybdenum in combination with a metal from the group consisting of cobalt, iron and nickel in an oxide form with an alkali metal promoter.

EPA 0119609 discloses an alkali-promoted molybdenum disulfide catalyst that is useful for producing aliphatic alcohols from synthesis gas.

US-A-4824869 discloses a process for converting synthesis gas into alcohols employing a catalyst comprising (a) an oxide support, (b) molybdenum, tungsten or rhenium oxide, (c) iron, cobalt, or nickel oxide, and (d) an alkali or alkaline earth metal promoter, which has been further treated with various nitrogen compounds.

A novel process to produce mixed alcohols comprises reacting mixtures of hydrogen and carbon monoxide in a slurry-phase system. A significant increase in the amount of $C_2$-$C_6$ aliphatic alcohols can be achieved in the slurry-phase when compared to operating a fixed-bed reactor at the same conditions.

In accordance with this invention, a proprietary mixed alcohols catalyst is evaluated in a modified Berty-type reactor in which internals have been replaced with special baffles and a stirrer designed to permit satisfactory catalyst suspension. In a typical experiment, the catalyst is reduced in-situ under hydrogen while suspended in an inert mineral oil.

Preferred reduction conditions comprise 4 to 16 hours at temperatures from 300 to 500°C under hydrogen pressures from 15 to 3000 psig (0.2 to 20.8 MPa). Still more preferred reduction conditions comprise about 8 hours at a temperature of about 400°C under about 1500 psig of hydrogen (10.4 MPa).

The carbon monoxide and hydrogen employed to form the lower aliphatic alcohols in this process can be provided from any available source. One particularly useful source is synthesis gas produced in the gasification of hydrocarbonaceous materials such as coals and biomass. An effective gasification process is described in U.S. 3,544,291 wherein a hydrocarbonaceous fuel is partially oxidized with a free oxygen-containing gas in a gas generator. In general, the mole ratio of hydrogen to carbon monoxide employed in this process should range from about 0.1 to 50 moles of hydrogen per mole of carbon monoxide with the

2

preferred ratio being from about 0.5 to 20 moles of hydrogen per mole of carbon monoxide.

The reaction conditions for effecting the conversion of the carbon monoxide-hydrogen feed into lower aliphatic alcohols employing the prescribed catalyst of the invention includes a reaction temperature ranging from about 240 to about 340°C with a more preferred temperature range being from about 255 to about 325°C and the most preferred temperature range being from about 270 to 310°C. The effective pressure range for this process is from about 3.4 MPa (500 psi) to about 17 MPa (2500 psi).

The space velocity employed to effect the conversion of carbon monoxide and hydrogen over the prescribed catalyst to the aliphatic alcohols is important. In general, the space velocity, that is the volume of gas passed through a given volume of catalyst per hour expressed as GHSV(hr$^{-1}$), must be at least 1000. A preferred range is from about 5000 to about 50,000. A highly effective process is realized when the space velocity employed ranges from 10,000 to 30,000 hr$^{-1}$.

The weight of catalyst suspended in the mineral oil is also important since it determines the viscosity of the slurry as well as the relative activity per unit of reactor volume. The effective range of slurry solids for this process is from 5 to 40 wt.% with a preferred range of slurry solids being from 10 to 25 wt.%.

The practice of this invention is described in the following Examples. The catalyst used in the Examples is that according to Example 3 of US-A-4824869, and comprises 7.0% of Mo, 1.32% of Co, 5.45% of K and 0.21% N, the latter resulting from pre-treatment of a molybdenum-containing alumina support with melamine.

## EXAMPLE I

In this Example, the reaction was carried out in a 1-liter Berty-type reactor modified as previously described. The reaction was carried out using 30 grams of catalyst suspended in 265 grams of Kaydol mineral oil. Kaydol is manufactured by Witco and is a 62:38 mixture of paraffinic and naphthenic components with an IBP of 732°C at 100 atmospheres (10.1 MPa) and a viscosity of 64-70 cst at 40°C. The catalyst was reduced in-situ under 1500 psig (10.4 MPa) of hydrogen at 350°C for 8 hours. Other run parameters were as follows:

$H_2/CO = 2/1$, T = 285°C, P = 1500 psig, (10.4 MPa) GHSV = 10,000 hr$^{-1}$, Stirrer RPM = 800 and slurry solids = 10 wt.%

The product emerging from the stainless steel reactor was then sent through a condenser which liquefied the alcohol and water products. The resulting liquid was analysed by gas chromatography. The alcohol productivity expressed as grams of alcohol per gram of catalyst per hour (g/g-hr) and the alcohol selectivity expressed as %C on a $CO_2$-free basis are reported on Table I, Run #5. Also reported are the wt.% of higher alcohols present in the liquid product (%HA) and the corresponding $C_{\frac{1}{2}}OH/C_1OH$ ratio.

Comparison of the results from Run #5 with performance of the same catalyst in a fixed bed reactor operating at $H_2/CO = 2/1$ is shown in Table I. As can be observed, and increase is obtained in $C_2 + OH/C_1OH$ from 0.29 in a fixed bed reactor to 0.94 in a slurry reactor operating at the same conditions. This corresponds to an increase from 22 to 48 wt.% in the relative amount of higher alcohols. A further increase in $C_2 + OH/C_1OH$ to 1.04 (51 wt.% higher alcohols) is achieved at the same experimental conditions by lowering the $H_2/CO$ ratio from 2/1 to 1/1 (Tale I, Run #6).

## EXAMPLE II

In this example, Witco-70 was used as the inert liquid medium, This oil is a 65:35 paraffinic:napthenic mixture, with an IBP of 632°C at 100 atmospheres (10.1 MPa) and a viscosity of 11-14 cst at 40°C. All other run conditions were identical to those of Example I. Selectivity, productivity, percentage of higher alcohols and $C_{\frac{1}{2}}OH/C_1OH$ ratio are given in Table I, Run #7. As can be determined, a $C_{\frac{1}{2}}OH/C_1OH$ ratio of 0.94 is obtained at these conditions, with a further increase to 1.05 achieved by lowering the $H_2/CO$ ratio from 2/1 to 1/1 (Table I, Run #8).

The increase in $C_{\frac{1}{2}}OH/C_1OH$ ratio reported in Examples I and II for a slurry-phase system when, compared to a fixed bed reactor, is probably due to solubility (CO has greater solubility than $H_2$ in the mineral oils) and/or mass transport effects. Differences in product residence times, due to backmixing in the slurry system, could also play a role in shifting the alcohols distribution to higher Cn values. This increase in $C_{\frac{1}{2}}OH/C_1OH$ ratio is a key element in-making the process economically attractive. Furthermore, the increased concentration of co-solvent alcohols will improve the water tolerance of alcohol mixtures when blended into gasoline.

Results of a factorial analysis to determine the effects of $H_2/CO$ ratio (R), stirrer RPM (F) and oil type (O), on $C_2 + OH/C_1OH$ and alcohol selectivity obtained in a slurry system are presented in Table II. Calculations have been made to evaluate the main effects, as well as two and three-factor interactions.

From Table II, the only significant effect on alcohol selectivity results from varying the $H_2/CO$ ratio, while the apparent effects remaining are generated by noise. It is observed that decreasing the $H_2/CO$ ratio from 2/1 to 1/1 results in a corresponding increase in selectivity of 5 percent. This effect is not observed in fixed bed reactors and suggests that, for slurry systems, lower concentrations of surface hydrogen might improve selectivity.

With respect to possible effects on the $C_2 + OH/C_1OH$ ratio, it is concluded from Table II that significant increases can be achieved using Kaydol oil and operating at $H_2/CO = 1$ and 800 rpm. However, since all three variables show evidence of two-factor interactions, their effects should be considered jointly.

An F*O interaction exists because changing the oil from Witco-70 to Kaydol results in an average $C_2 + OH/C_1OH$ increase of 0.15 when operating at 600 rpm but has a negligible effect at 800 rpm. It appears that operating the stirrer at 800 rpm minimizes the mass transfer effects associated with the physical properties of the liquid medium.

An R*F interaction arises because decreasing the $H_2/CO$ ratio from 2/1 to 1/1 produces and average $C_2 + OH/C_1OH$ increase of 0.17 at 600 rpm but only 0.10 at 800 rpm.

From Table II, it can also be concluded that no interaction exists between $H_2/CO$ ratio and oil type, i.e., R*O = 0. This would suggest that the relative solubilities and mass transport properties of hydrogen and carbon monoxide are the same in Witco-70 and Kaydol.

TABLE I

| Run # | H2/CO | OIL TYPE | STIRRER RPM | SEL | PROD | % HA | RATIO |
|-------|-------|----------|-------------|-----|------|------|-------|
| 1 | 2/1 | KAYDOL | 600 | 67 | 0.24 | 46 | 0.85 |
| 2 | 1/1 | KAYDOL | 600 | 74 | 0.21 | 50 | 1.02 |
| 3 | 2/1 | WITCO-70 | 600 | 72 | 0.25 | 41 | 0.70 |
| 4 | 1/1 | WITCO-70 | 600 | 70 | 0.16 | 46 | 0.86 |
| 5 | 2/1 | KAYDOL | 800 | 70 | 0.20 | 48 | 0.94 |
| 6 | 1/1 | KAYDOL | 800 | 77 | 0.24 | 51 | 1.04 |
| 7 | 2/1 | WITCO-70 | 800 | 67 | 0.23 | 48 | 0.94 |
| 8 | 1/1 | WITCO-70 | 800 | 74 | 0.23 | 51 | 1.05 |
| | 2/1 | FIXED BED PERFORMANCE | | 81 | 0.36 | 22 | 0.29 |

EP 0 353 920 A2

## TABLE II

| CALCULATED EFFECTS FOR $2^3$ FACTORIAL DESIGN | | |
|---|---|---|
| | $C_2OH/C_1OH$ | SELECTIVITY |
| Main Effects | | |
| $H_2/CO$ (R) | -0.14 | -4.8 |
| Stirrer RPM (F) | +0.14 | +1.2 |
| Oil type (0) | +0.07 | +1.2 |
| Two-Factor Interactions | | |
| RxF | 0.04 | -2.2 |
| RxO | 0 | -2.2 |
| FxO | -0.08 | +1.8 |
| Three-Factor Interaction RxFxO | +0.01 | +2.2 |
| Estimated Standard Error | ±0.02 | ±3 |

## Claims

1. A method for preparing a mixture of lower aliphatic alcohols by reacting carbon monoxide and hydrogen in the presence of a mixed-alcohols catalyst, characterised in that the catalyst is suspended in an inert liquid medium.

2. A method according to Claim 1 characterised in that the inert liquid medium is oil having paraffinic and naphthenic components.

3. A method according to Claim 1 or 2 characterised in that the alcohol catalyst is reduced in-situ at a temperature of about 350°C for about 8 hours.

4. A method according to any one of Claims 1 to 3 characterised in that the hydrogen to carbon monoxide mole ratio is from 0.5 to 5.0.

5. A method according to any one of Claims 1 to 4 characterised in that the reaction temperature is from 270 to 310°C.

6. A method according to any one of Claims 1 to 5 characterised in that the reaction pressure is from 500 to 2500 psig (3.4 to 17 MPa).

7. A method according to any one of Claims 1 to 6 characterised in that the space velocity is from 5,000 to 15,000 hr$^{-1}$.

8. A method according to any one of Claims 1 to 7 characterised in that the catalyst is a slurry having a solid content from 10 to 25 wt.%.

5